# EUROPEAN PATENT APPLICATION

(11) **EP 2 502 559 A1**
(43) Date of publication of application: **26.09.2012**
(21) Application number: 10831341.2
(22) Date of filing: 19.11.2010
(51) Int. Cl.: A61B 5/107, A61B 5/08, G01B 13/10

(54) **INNER DIAMETER MEASURING DEVICE AND METHOD FOR PRIMING SAME**

(30) Priority: 20.11.2009 JP 2009264718
(71) Applicant: Sumitomo Bakelite Co., Ltd., Shinagawa-ku Tokyo 140-0002 (JP)
(72) Inventor: HASHIDO, Hiroaki, Akita-shi Akita 011-8510 (JP); IKEDA, Masao, Akita-shi Akita 011-8510 (JP)
(74) Representative: Vossius, Corinna
(86) International application number: PCT/JP2010/006792
(87) International publication number: WO 2011/061942

(57) **Abstract**

An inner diameter measurement instrument (100) includes a first channel (120) and a second channel (140) respectively communicating with a balloon (110), a fluid injection mechanism (130), a fluid lock mechanism (150), and an amount measurement unit (160). The fluid injection mechanism (130) injects an incompressible fluid into the balloon (110) through the first channel (120) or the second channel (140). The fluid lock mechanism (150) closes the first channel (120) or the second channel (140). The amount measurement unit (160) measures the amount of an incompressible measurement fluid additionally injected after the balloon (110), the first channel (120), and the second channel (140) are filled with the fluid and the fluid lock mechanism (150) closes one of the first channel (120) and the second channel (140), by the fluid injection mechanism (130) through the other of the first channel (120) and the second channel (140).

## Description

### [TECHNICAL FIELD]

The present invention relates to an inner diameter measurement instrument to be used for measuring an inner diameter of a conduit, and more particularly to an inner diameter measurement instrument suitable for measuring an inner diameter of a bronchus in pneumonectasia treatment, and to a priming method for the inner diameter measurement instrument.

### [BACKGROUND ART]

Treatment remedies of pneumonectasia that are currently practiced include placing a valve in the bronchus of the patient. The valve employed for such a purpose has, for example, a structure that has an umbrella and a balloon integrally attached in the umbrella. The bronchus to be treated can be blocked by opening and closing the valve like an umbrella.

Various instruments that block the bronchus as above have thus far been proposed (for example, see patent documents 1, 2). In addition, various types of catheters that employ a one-way valve or the like have been proposed (for example, see patent documents 3 to 6).

Further, inner diameter measurement instruments capable of accurately measuring the inner diameter of the measuring object irrespective of the size thereof have also been proposed. One of such inner diameter measurement instruments, to be inserted in the lumen to measure the inner diameter thereof, includes a tubular sheath to be inserted in the lumen, a balloon attached to a distal end portion of the sheath so as to be expanded upon supplying a fluid into the balloon, a linear index member having a first end portion fixed to the distal end portion of the sheath or the balloon so that a second end portion moves toward the distal end portion of the sheath following up the expansion of the balloon, and a measurement unit provided on the proximal end portion of the sheath. The measurement unit is capable of confirming the position of the second end portion of the index member (for example, see patent document 7).

### [RELATED DOCUMENT]

### [PATENT DOCUMENT]

[Patent document 1] JP-T-No.2005-505355
[Patent document 2] JP-A-No.2004-024864
[Patent document 3] JP-U-No.S63-022939
[Patent document 4] JP-A-No.H03-109065
[Patent document 5] JP-A-No.H05-049597
[Patent document 6] JP-A-No.H08-019605
[Patent document 7] JP-A-No.2009-165608

### [DISCLOSURE OF THE INVENTION]

### [PROBLEM TO BE SOLVED BY THE INVENTION]

Since the inner diameter of the bronchus is uneven, valves of different sizes are employed depending on the size of the bronchus to be treated. Accordingly, the inner diameter of the portion of the bronchus to be blocked has to be confirmed in advance, to select an optimum valve for treatment.

The present invention has been accomplished in view of the foregoing problem, and provides an inner diameter measurement instrument that allows the inner diameter of the conduit of the bronchus to be easily measured, and a priming method for operating such an inner diameter measurement instrument.

### [SOLUTION TO PROBLEM]

Accordingly, the present invention provides an inner diameter measurement instrument to be used for measuring an inner diameter of a conduit. The inner diameter measurement instrument includes a balloon element to be inserted in the conduit to a position where the inner diameter is to be measured, a first channel member and a second channel member respectively communicating with the balloon element, a fluid injection mechanism that injects an incompressible fluid into the balloon element through the first channel member or the second channel member, a fluid lock mechanism that closes one of the first channel member and the second channel member, and an amount measurement unit that measures an amount of an incompressible measurement fluid additionally injected by the fluid injection mechanism through the other of the first channel member and the second channel member, after the balloon element, the first channel member, and the second channel member are filled with the fluid and the fluid lock mechanism closes the one of the first channel member and the second channel member.

With such a configuration, the incompressible measurement fluid is additionally injected after the inner diameter measurement instrument is filled with the incompressible fluid, in other words in a primed state. Therefore, the amount of the additionally injected measurement fluid accurately corresponds to the expansion of the volume of the balloon element.

In the foregoing inner diameter measurement instrument, the fluid injection mechanism may inject the fluid and the measurement fluid into the first channel member through a proximal end portion thereof; the fluid lock mechanism may openably close a proximal end portion of the second channel member filled with the fluid as are the first channel member and the balloon element; and the amount measurement unit may measure the amount of the measurement fluid injected into the first channel member by the fluid injection mechanism with the fluid lock mechanism set in a closed state. Hereinafter, the inner diameter measurement instrument thus configured may be referred to as a first inner diameter measurement instrument.
With the first inner diameter measurement instrument, the amount of the fluid corresponding to the inner diameter of the conduit is measured when the inner diameter measurement instrument is in the primed state, i.e. , filled with the incompressible fluid. The priming process of filling the inner diameter measurement instrument with the fluid and the measurement of the inner diameter are performed by the fluid injection mechanism.

In the foregoing inner diameter measurement instrument, the fluid injection mechanism may include a first injection mechanism that injects the fluid into the first channel member through the proximal end portion thereof and a second injection mechanism removably connected to the proximal end portion of the second channel member so as to inject the measurement fluid thereinto. The fluid lock mechanism may include a first block mechanism that inhibits a reverse flow of the fluid into the first injection mechanism from the first channel member filled with the fluid as are the second channel member and the balloon element; the second injection mechanism may inject the measurement fluid through the proximal end portion of the second channel member with the first block mechanism set to inhibit the reverse flow; and the amount measurement unit may measure the amount of the measurement fluid injected by the second injection mechanism into the second channel member. Hereinafter, the inner diameter measurement instrument thus configured may be referred to as a second inner diameter measurement instrument.
With the second inner diameter measurement instrument, the amount of the fluid corresponding to the inner diameter of the conduit is measured when the inner diameter measurement instrument is in the primed state, i.e., filled with the incompressible fluid. The priming process of filling the inner diameter measurement instrument with the fluid is performed by the first injection mechanism, and the inner diameter measurement is performed by the second injection mechanism.

In the foregoing inner diameter measurement instrument, the fluid injection mechanism may include the first injection mechanism that injects the fluid into the first channel member through the proximal end portion thereof and a second injection mechanism that injects the measurement fluid through the proximal end portion of the first channel member. The fluid lock mechanism may openably close the proximal end portion of the second channel member filled with the fluid as are the balloon element and the first channel member, and may inhibit the reverse flow of the fluid into the first injection mechanism from the first channel member filled with the fluid as are the balloon element and the second channel member; the second injection mechanism may inject the measurement fluid through the proximal end portion of the first channel member with a state in which the reverse flow into the first injection mechanism is inhibited; and the amount measurement unit may measure the amount of the measurement fluid injected into the first channel member by the second injection mechanism. Hereinafter, the inner diameter measurement instrument thus configured may be referred to as a third inner diameter measurement instrument.
With the second inner diameter measurement instrument, the amount of the fluid corresponding to the inner diameter of the conduit is measured when the inner diameter measurement instrument is in the primed state, i.e. , filled with the incompressible fluid. The priming process of filling the inner diameter measurement instrument with the fluid is performed by the first injection mechanism, and the inner diameter measurement is performed by the second injection mechanism. Such an arrangement eliminates the need to mount or remove the members during the entire measurement work, thereby allowing the inner diameter measurement to be easily and hygienically performed.

The foregoing inner diameter measurement instrument may further include a reverse flow block mechanism provided in a flow path of the measurement fluid so as to allow the measurement fluid to flow from the fluid injection mechanism toward the balloon element, and so as to inhibit a reverse flow.

In the foregoing inner diameter measurement instrument, the reverse flow block mechanism may be manually operable so as to allow the reverse flow of the measurement fluid.

Further, the reverse flow block mechanism may include a one-way valve that can be displaced between an open state that allows the measurement fluid to be introduced and a closed state that inhibits the reverse flow, and a pair of pressers provided on the respective sides of the one-way valve, and the one-way valve may be opened by pressing the pressers so as to allow the reverse flow of the measurement fluid.

Further, the fluid injection mechanism may inject the measurement fluid at smaller injection rate than that of the fluid.

Further, in the foregoing inner diameter measurement instrument, the fluid injection mechanism may include a first syringe that injects the fluid and a second syringe that injects the measurement fluid, and a scale increment of the second syringe may be smaller than a scale increment of the first syringe.

Still further, in the foregoing inner diameter measurement instrument, the fluid lock mechanism may include a valve mechanism having a valve element that closes the proximal end portion of the second channel member and an elastic member that biases the valve element to the closed state, and a clamp removably attached to the valve mechanism so as to set the valve element in an open state.

Still further, in the foregoing inner diameter measurement instrument, the fluid lock mechanism may include a manual valve mechanism that opens and closes communication between the first channel member and the first injection mechanism, or a one-way valve that allows the fluid to be introduced from the first injection mechanism into the first channel member and inhibits the reverse flow.

Still further, the inner diameter measurement instrument may further include an inner diameter indicator that shows the inner diameter of the conduit corresponding to an outer diameter of the expanded balloon element obtained on the basis of the measured amount of the measurement fluid.

Still further, the inner diameter measurement instrument may further include a first opening formed at a distal end portion of the first channel member and a second opening formed at a distal end portion of the second channel member. The first opening and the second opening may be located inside the balloon element so as to communicate with each other through the balloon element, and
one of the first opening and the second opening, which is formed in one of the first channel member through which the fluid is injected by the fluid injection mechanism into the balloon element and the second channel member through which the fluid is injected by the fluid injection mechanism into the balloon element, may be located at a more distal position than the other formed in the other of the first channel member and the second channel member.

Still further, in the foregoing inner diameter measurement instrument, a region of the first channel member and the second channel member, on a further distal side of the one of the first opening and the second opening located at the more distal position than the other, may be filled with a resin material which is adjacent to the one of the first opening and the second opening.

The present invention also provides a priming method of an inner diameter measurement instrument in which an incompressible fluid is introduced into a balloon element. The inner diameter measurement instrument is configured to measure an inner diameter of a conduit by using the balloon element.
The priming method includes injecting the fluid into the balloon element through a first channel member among the first channel member and a second channel member respectively communicating with the balloon element, causing the introduced fluid to flow out from the second channel member, and closing the second channel member when the balloon element, the first channel member, and the second channel member are filled with the fluid.

The foregoing priming method may further include injecting the fluid into the first channel member through a proximal end portion thereof until the fluid flows out through a proximal end portion of the second channel member, so as to fill the first channel member, the balloon element, and the second channel member with the fluid; and closing the proximal end portion of the second channel member filled with the fluid as are the first channel member and the balloon element.

The foregoing priming method may include filling the first channel member, the balloon element, and the second channel member with the fluid by injecting the fluid through the proximal end portion of the first channel member until the fluid flows out through the proximal end portion of the second channel member, and inhibiting a reverse flow of the fluid through the proximal end portion of the first channel member filled with the fluid as are the balloon element and the second channel member.

The foregoing priming method may include, in the case where the proximal end portion of the first channel member is branched into a plurality of flow paths, injecting the fluid into the balloon element through one of the flow paths so as to fill the first channel member, the balloon element, and the second channel member with the fluid, inhibiting a reverse flow of the fluid through the one of the flow paths of the first channel member filled with the fluid as are the balloon element and the second channel member, and closing the proximal end portion of the second channel member filled with the fluid as are the balloon element and the first channel member.

It is to be noted that the constituents of the present invention do not necessarily have to be individually independent, but may be configured such that a plurality of constituents constitutes a single member, that a constituent is composed of a plurality of members, that a constituent is a part of another constituent, that a part of a constituent and a part of another constituent overlap, and so forth.

### [ADVANTAGEOUS EFFECT OF THE INVENTION]

With the inner diameter measurement instrument according to the present invention, the amount of the measurement fluid injected by the fluid injection mechanism corresponds to the expansion of the volume of the balloon element. Therefore, the inner diameter of the conduit can be accurately measured on the basis of the amount of the measurement fluid measured by the amount measurement unit.

With the first inner diameter measurement instrument in particular, the incompressible fluid is injected by the fluid injection mechanism through the proximal end portion of the first channel member communicating with the balloon element inserted in the conduit to a position where the inner diameter is to be measured. The fluid overflows through the proximal end portion of the second channel member communicating with the balloon element, and the proximal end portion of the second channel member, filled with the fluid as are the balloon element and the first channel member, is closed by the fluid lock mechanism. Then the amount of the measurement fluid, injected into the first channel member by the fluid injection mechanism with the fluid lock mechanism set in the closed state, is measured by the amount measurement unit. Upon additionally injecting the measurement fluid into the first channel member in the primed state in which the inner diameter measurement instrument is filled with the incompressible fluid, the internal pressure of the balloon element is increased. Accordingly, the balloon element is expanded until the diameter thereof becomes equal to the inner diameter of the conduit, at which point the inner diameter of the conduit can be measured. Thus, a compressible fluid such as air is not involved in the priming process and the inner diameter measurement, and therefore the balloon element can be prevented from abruptly expanding, which allows the inner diameter of the conduit to be measured safely and accurately.

In the case of the second inner diameter measurement instrument, the incompressible fluid is injected by the first injection mechanism through the proximal end portion of the first channel member communicating with the balloon element inserted in the conduit to the position where the inner diameter is to be measured. The fluid is injected until it overflows through the proximal end portion of the second channel member communicating with the balloon element, and the first block mechanism inhibits the reverse flow of the fluid into the first injection mechanism from the first channel member, filled with the fluid as are the balloon element and the second channel member. Then the second injection mechanism is removably connected to the proximal end portion of the second channel member, in which the reverse flow is inhibited by the first block mechanism, and the amount of the measurement fluid injected by the second injection mechanism into the second channel member is measured by the amount measurement unit. Upon additionally injecting the measurement fluid into the second channel member in the primed state in which the inner diameter measurement instrument is filled with the incompressible fluid, the balloon element is expanded so as to enable the measurement of the inner diameter of the conduit. Thus, a compressible fluid such as air is not involved in the priming process and the inner diameter measurement, and therefore the balloon element can be prevented from abruptly expanding, which allows the inner diameter of the conduit to be measured safely and accurately. In addition, the priming process of filling the inner diameter measurement instrument with the fluid can be performed by the first injection mechanism and the inner diameter measurement can be performed by the second injection mechanism. Such an arrangement allows the inner diameter measurement to be directly performed by the second injection mechanism.

In the case of the third inner diameter measurement instrument, the incompressible fluid is injected by the first injection mechanism through the proximal end portion of the first channel member communicating with the balloon element inserted in the conduit to the position where the inner diameter is to be measured. The fluid overflows through the proximal end portion of the second channel member communicating with the balloon element, and the fluid lock mechanism inhibits the reverse flow of the fluid into the first injection mechanism from the first channel member filled with the fluid as are the balloon element and the second channel member. Then the proximal end portion of the second channel member, filled with the fluid as are the balloon element and the first channel member, is closed by the fluid lock mechanism. Then the measurement fluid is injected by the second injection mechanism into the proximal end portion of the first channel member, in which the reverse flow into the first injection mechanism is inhibited, and the amount of the measurement fluid injected by the second injection mechanism into the first channel member is measured by the amount measurement unit. Upon additionally injecting the measurement fluid into the first channel member in the primed state in which the inner diameter measurement instrument is filled with the incompressible fluid, the balloon element is expanded so as to enable the measurement of the inner diameter of the conduit. Thus, a compressible fluid such as air is not involved in the priming process and the inner diameter measurement, and therefore the balloon element can be prevented from abruptly expanding, which allows the inner diameter of the conduit to be measured safely and accurately. In addition, the priming process of filling the inner diameter measurement instrument with the fluid can be performed by the first injection mechanism and the inner diameter measurement can be performed by the second injection mechanism. Such an arrangement allows the inner diameter measurement to be directly performed by the second injection mechanism. Further, since there is no need to mount or remove the members during the entire measurement work, the inner diameter measurement can be easily and hygienically performed.

### [BRIEF DESCRIPTION OF THE DRAWINGS]

The above and other objects, features and advantages will become more apparent through the preferred embodiments described hereunder and the accompanying drawings.

Fig. 1 is a schematic drawing showing an appearance of an inner diameter measurement instrument according to a first embodiment of the present invention.
Fig. 2 is a schematic vertical cross-sectional view showing an inner structure of an essential part of the inner diameter measurement instrument.
Fig. 3 is a flowchart showing a priming method and an inner diameter measurement method performed by the inner diameter measurement instrument according to the first embodiment.
Fig. 4 is a characteristic diagram showing a relationship between an injection amount of a fluid and an expanded outer diameter of a balloon element, in the respective cases where the fluid is air and pure water.
Figs. 5(a) and 5(b) are a plan view and a left side view respectively, showing a reverse flow block mechanism.
Fig. 6 is a schematic drawing showing an appearance of an inner diameter measurement instrument according to a second embodiment of the present invention.
Fig. 7 is a flowchart showing a priming method and an inner diameter measurement method performed by the inner diameter measurement instrument according to the second embodiment.
Fig. 8 is a schematic drawing showing an appearance of an inner diameter measurement instrument according to a third embodiment of the present invention.
Fig. 9 is a flowchart showing a priming method and an inner diameter measurement method performed by the inner diameter measurement instrument according to the third embodiment.
Figs. 10(a) and 10(b) are schematic drawings showing appearances of a valve mechanism and a clamp serving as a fluid lock mechanism according to a first variation.
Fig. 11 is a schematic vertical cross-sectional view showing an inner structure of an essential part of an inner diameter measurement instrument according to a second variation.

### [DESCRIPTION OF EMBODIMENTS]

### First Embodiment: First inner diameter measurement instrument

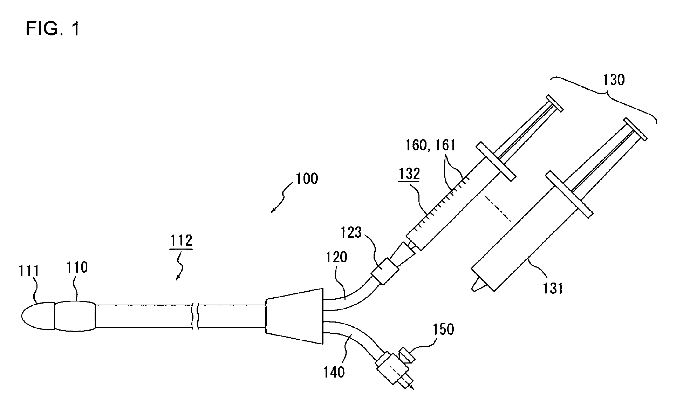

Referring to Figs. 1 to 4, a first embodiment of the present invention will be described hereunder. An inner diameter measurement instrument 100 according to this embodiment is intended for use in treatment of pneumonectasia, to measure an inner diameter of a given position of a conduit (not shown) such as a bronchus.

The inner diameter measurement instrument 100 according to this embodiment includes a balloon element 110, a first channel member 120, a second channel member 140, a fluid injection mechanism 130, a fluid lock mechanism 150, and an amount measurement unit 160. The balloon element 110 is inserted in the conduit to a position where the inner diameter is to be measured. The first channel member 120 and the second channel member 140 respectively communicate with the balloon element 110. The fluid injection mechanism 130 injects an incompressible fluid into the balloon element 110 through one of the first channel member 120 and the second channel member 140 (first channel member 120 in this embodiment). The fluid lock mechanism 150 closes one of the first channel member 120 and the second channel member 140 (second channel member 140 in this embodiment). The amount measurement unit 160 measures the amount of an incompressible measurement fluid additionally injected after the balloon element 110, the first channel member 120, and the second channel member 140 are filled with the fluid and the fluid lock mechanism 150 closes one of the first channel member 120 and the second channel member 140 (second channel member 140 in this embodiment), by the fluid injection mechanism 130 through the other of the first channel member 120 and the second channel member 140 (first channel member 120 in this embodiment).

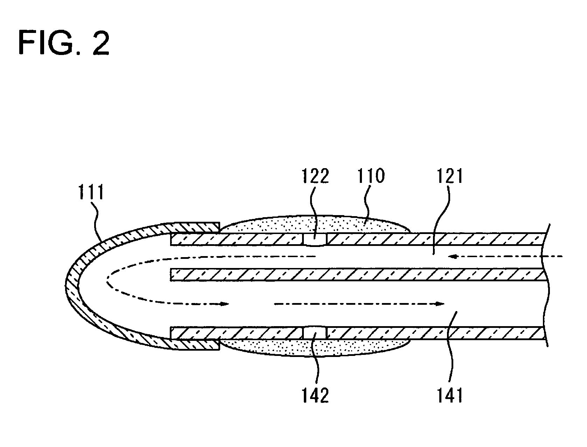

In the inner diameter measurement instrument 100 according to this embodiment, the first channel member 120 and the second channel member 140 communicate with the balloon element 110, as shown in Figs. 1 and 2.
The fluid injection mechanism 130 injects the incompressible fluid and the measurement fluid into the first channel member 120 through a proximal end portion thereof. The fluid lock mechanism 150 according to this embodiment openably closes the proximal end portion of the second channel member 140 filled with the fluid as are the first channel member 120 and the balloon element 110. The amount measurement unit 160 measures the amount of the measurement fluid injected by the fluid injection mechanism 130 into the first channel member 120, with the fluid lock mechanism 150 set in the closed state.

More specifically, the balloon element 110 is formed so as to have a diameter sufficiently smaller than the conduit to be measured, in an initial state before being expanded. The first channel member 120 and the second channel member 140 are, as shown in Figs. 1 and 2, prolonged portions to be inserted in a human body (not shown) and unified so as to constitute a balloon catheter 112, which includes therein an inflow path 121 and an outflow path 141. The first channel member 120 communicates with the inflow path 121 of the balloon catheter 112 via a connector. The second channel member 140 communicates with the outflow path 141 of the balloon catheter 112 via a connector.

As shown in Fig. 2, in the inner diameter measurement instrument 100 according to this embodiment the respective distal end portions of the inflow path 121 of the first channel member 120 and the outflow path 141 of the second channel member 140 are open and covered with, for example, a cap 111.

Accordingly, the inflow path 121 and the outflow path 141 communicate with each other inside the cap 111. In addition, the inflow path 121 of the first channel member 120 and the outflow path 141 of the second channel member 140 communicate with each other also inside the balloon element 110, through orifices 122, 142.

In the inner diameter measurement instrument 100 according to this embodiment, the inflow path 121 of the first channel member 120 is formed in a smaller diameter than the outflow path 141 of the second channel member 140 as shown in Fig. 2, so that the fluid injection mechanism 130 for press-injecting the fluid into the balloon element 110 can be operated with a smaller driving force.

From another viewpoint, in the inner diameter measurement instrument 100 according to this embodiment, the outflow path 141 of the second channel member 140 is formed in a larger diameter than the inflow path 121 of the first channel member 120, so as to effectively prevent air bubbles from residing inside in the priming process.

The fluid injection mechanism 130 is constituted of what is known as syringe, which can be manually operated so as to inject the fluid into the first channel member 120. The fluid may be, for example, pure water or physiological saline.

The fluid injection mechanism 130 serves to inject the fluid into the balloon element 110 under ambient pressure (atmospheric pressure). The fluid injection mechanism 130 also serves to additionally press-inject the measurement fluid toward the balloon element 110 to thereby expand the balloon element 110, with the inner diameter measurement instrument 100 closed so as to prevent the fluid from flowing out.

In this embodiment, the "fluid" refers to the liquid preliminarily introduced in the first channel member 120, the balloon element 110, and the second channel member 140 (hereinafter collectively referred to as "measurement system" where appropriate) under ambient pressure. Hereafter, filling the measurement system with the fluid under ambient pressure in advance of the inner diameter measurement of the conduit may be referred to as "preliminary filling" or "priming".
Further, the "measurement fluid" refers to the liquid additionally introduced into the measurement system already filled with the fluid, so as to elastically expand the balloon element 110. The fluid and the measurement fluid are mixed with each other upon being introduced into the measurement system. Hereafter, injecting the measurement fluid into the preliminarily filled measurement system may be referred to as "additional injection".

The fluid and the measurement fluid may be introduced into the measurement system from a common syringe. In other words, the fluid and the measurement fluid may be the same liquid contained in a single syringe without distinction from each other. Alternatively, the fluid and the measurement fluid may be separately contained in different syringes so as to be independently introduced into the measurement system.

In addition, "injecting the measurement fluid into the balloon element 110" means both introducing the measurement fluid inside the balloon element 110 through the first channel member 120 or the second channel member 140, and injecting the measurement fluid into the first channel member 120 or the second channel member 140 with the measurement system preliminarily filled with the fluid, to thereby squeeze the fluid into the balloon element 110.

Here, the "fluid" and the "measurement fluid" are both incompressible fluid, and may be the same or dissimilar from each other. For example, physiological saline may be employed as the fluid and pure water may be employed as the measurement fluid, and alternatively either pure water or physiological saline may be employed as the fluid and the measurement fluid.

The balloon element 110 assumes a generally circular shape upon being expanded, and the expanded shape is reproducible. The correlation between the amount of the measurement fluid additionally introduced into the balloon element 110 and the outer diameter of the balloon element 110 is defined in advance. Accordingly, measuring the amount of the measurement fluid additionally introduced into the measurement system preliminarily filled with the fluid leads to determining the outer diameter of the balloon element 110. In the process of expanding the balloon element 110 located inside the conduit, the pressure necessary for injecting the measurement fluid discontinuously increases at the point where the outer diameter of the balloon element 110 becomes equal to the inner diameter of the conduit. Accordingly, the operator can calculate the inner diameter of the conduit by measuring the amount of the measurement fluid additionally injected up to that point.

The fluid injection mechanism 130 may be constituted of a single injection mechanism (for example, a syringe) or may include a mechanism for preliminarily introducing the fluid and another mechanism for additionally injecting the measurement fluid. The fluid injection mechanism 130 according to this embodiment is composed of a combination of a first syringe 131 (first injection mechanism) for preliminarily introducing the fluid and a second syringe 132 (second injection mechanism) for additionally injecting the measurement fluid.
The first syringe 131 and the second syringe 132 are selectively attached to a reverse flow block mechanism 123 to be subsequently described.

Thus, the inner diameter measurement instrument 100 according to this embodiment includes the first syringe 131 for injecting the fluid and the second syringe 132 for injecting the measurement fluid. The scale increment of the second syringe 132 is smaller than that of the first syringe 131. Therefore, the inner diameter of the conduit can be precisely measured.
It is not mandatory that the first syringe 131 be provided with the scale. In this case, the increment of the scale of the first syringe 131 corresponds to the entire capacity of the first syringe 131. The second syringe 132 may have a smaller capacity than the first syringe 131.

The fluid injection mechanism 130 is configured so as to inject the measurement fluid at smaller injection rate than that of the fluid. More specifically, the inner diameter of the second syringe 132 is smaller than that of the first syringe 131, and hence an amount dispensed from the second syringe 132 is smaller than an amount dispensed from the first syringe 131, in the case where the piston stroke is the same. Such a configuration allows the fluid to be preliminarily introduced quickly, and the measurement fluid for measuring the inner diameter of the conduit to be additionally injected precisely.

The fluid lock mechanism 150 serves to prevent the fluid preliminarily filled in the measurement system from flowing out therefrom. In other words, the fluid lock mechanism 150 closes the flow path of the measurement system of the inner diameter measurement instrument 100 other than the flow path through which the measurement fluid is introduced (first channel member 120 in this embodiment). The fluid lock mechanism 150 maintains the inner diameter measurement instrument 100 in the state where the measurement system is preliminarily filled with the fluid, in other words the state ready for measuring the inner diameter of the conduit by additionally injecting the measurement fluid.

Since the same channel (first channel member 120) is employed in common for preliminarily introducing the fluid and additionally injecting the measurement fluid in the inner diameter measurement instrument 100 according to this embodiment, the fluid lock mechanism 150 is provided in the other channel (second channel member 140). In contrast, in the case where different channels are employed for preliminarily introducing the fluid (first channel member 120) and additionally injecting the measurement fluid (second channel member 140) as will be subsequently described in a second embodiment, the fluid lock mechanism 150 may be provided in the channel for preliminarily introducing the fluid, so as to prevent the fluid from flowing out (flowing backward).

The fluid lock mechanism 150 according to this embodiment is constituted of a known manual valve mechanism, and can be manually operated so as to openably close the proximal end portion of the second channel member 140.

The amount measurement unit 160 is employed for measuring the amount of the measurement fluid that has been additionally introduced. Among various methods of measuring the amount of the measurement fluid, this embodiment adopts a scale 161 marked on the second syringe 132 (second injection mechanism). Thus, in the inner diameter measurement instrument 100 according to this embodiment the first syringe 131 of the larger capacity and the second syringe 132 of the smaller capacity are replaceably attached to the first channel member 120, so that the first syringe 131 is utilized for the priming process to be subsequently described and the measurement of the inner diameter of the conduit can be performed by measuring the injected amount of the measurement fluid by visually reading the syringe scale (amount measurement unit 160). Alternatively, a positive displacement flow meter (not shown) may be provided in the flow path of the first channel member 120, so as to work as the amount measurement unit 160.

In the inner diameter measurement instrument 100 according to this embodiment, the first syringe 131 is replaced with the second syringe 132 when the fluid lock mechanism 150 closes the proximal end portion of the second channel member 140. Accordingly, it is preferable to provide in the first channel member 120 a mechanism that inhibits the reverse flow of the introduced fluid, so as to prevent the fluid from flowing out from the measurement system during the replacement of the syringes.
In addition, in the case where the measurement fluid injected from the second syringe 132 into the measurement system flows backward into the second syringe 132 while measuring the inner diameter of the conduit, the amount of the measurement fluid may be underestimated and hence the inner diameter of the conduit may be erroneously determined to a lower side. From such a viewpoint also, it is preferable to provide a mechanism that prevents the reverse flow of the introduced fluid (measurement fluid), in the first channel member 120.

For the aforementioned reason, the inner diameter measurement instrument 100 according to this embodiment includes the reverse flow block mechanism 123 provided in the flow path of the measurement fluid so as to allow the measurement fluid flowing toward the balloon element 110 from the fluid injection mechanism 130 to pass therethrough, and so as to prevent the reverse flow. More specifically, the reverse flow block mechanism 123 includes a one-way valve that only permits the flow toward the balloon element 110 through the first channel member 120.

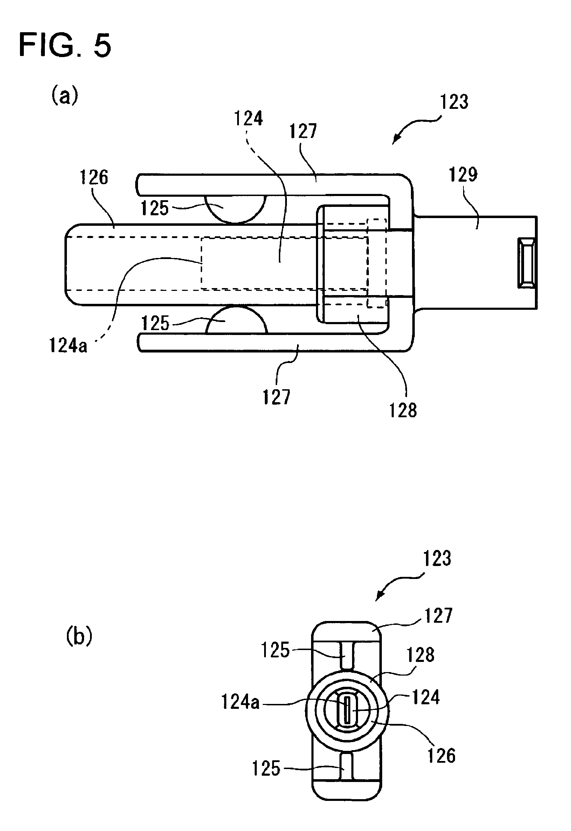

Figs. 5(a) and 5(b) are a plan view and a left side view respectively, showing the reverse flow block mechanism 123 according to this embodiment. Referring to Fig. 5(a), the fluid injection mechanism 130 (first syringe 131 and second syringe 132) is connected to the right side of the reverse flow block mechanism 123, and the first channel member 120 and the balloon element 110 are connected to the left side.

The reverse flow block mechanism 123 according to this embodiment can be manually operated so as to allow the measurement fluid (fluid) to flow backward. More specifically, the reverse flow block mechanism 123 includes a one-way valve 124 that can be displaced between an open state that allows the measurement fluid to be introduced and a closed state that inhibits the reverse flow, and a pair of pressers 125 provided on the respective sides of the one-way valve 124, so that the one-way valve 124 can be opened by pressing the pressers 125 and the measurement fluid is allowed to flow backward.

The one-way valve 124 is accommodated in a cylindrical housing 126. The outer diameter of the one-way valve 124 is generally the same as the inner diameter of the housing 126. An end portion of the housing 126 is supported by a fitting member 129. The fitting member 129 provides communication between the housing 126 and the fluid injection mechanism 130.
The fitting member 129 includes a pair of arms 127 extending along the housing 126 and an annular support base 128 into which the end portion of the housing 126 is fitted. Each of the pair of pressers 125 is formed so as to inwardly project from a distal portion of the corresponding arm 127. In this embodiment, the presser 125 has a semicircular disk shape.

The tip portion of the one-way valve 124 (open end 124a that regulates the reverse flow of the measurement fluid) and the pressers 125 are located in a generally central portion of the housing 126. The housing 126 and the one-way valve 124 are formed of a soft resin material. The fitting member 129 is formed of a hard resin material or a metal material.
The pair of pressers 125 according to this embodiment is located on the respective sides of the one-way valve 124. The expression "pressers 125 are located on the respective sides of the one-way valve 124" refers to the case where a plurality of pressers 125 is disposed so as to oppose the one-way valve 124, as well as the case where annular or semiannular pressers 125 are provided around the periphery of the one-way valve 124.

Various valve structure may be adopted as the one-way valve 124. The one-way valve 124 according to this embodiment is a duckbill valve that includes the open end 124a of a slit shape. The presser 125 is provided at the side of the slit direction of the open end 124a respectively.
The slit-shaped open end 124a is oriented such that the respective end portions thereof oppose the pressers 125. Thus, when the operator presses the pair of arms 127 of the fitting member 129, the pressers 125 squeeze the one-way valve 124 so as to flexurally deform the same, to thereby forcibly open up the open end 124a.
Accordingly, in the inner diameter measurement instrument 100 according to this embodiment, the measurement fluid (fluid) is allowed to flow backward into the fluid injection mechanism 130 from the first channel member 120 only when the operator activates the reverse flow block mechanism 123. When the second syringe 132 is connected to the reverse flow block mechanism 123 and the measurement fluid is additionally injected, the internal pressure in the measurement system is higher than the ambient pressure. Therefore, upon activating the reverse flow block mechanism 123, the measurement fluid flows backward into the second syringe 132 from the first channel member 120, and hence the expanded balloon element 110 restores the initial state.

The foregoing structure allows the operator to repeat as desired the additional injection of the measurement fluid from the second syringe 132 into the measurement system and the activation of the reverse flow block mechanism 123 to allow the measurement fluid to flow backward into the second syringe 132. Accordingly, even though the measurement fluid is excessively injected into the measurement system by improper handling of the second syringe 132, the balloon element 110 can be restored to the initial state by the simple manual operation of the reverse flow block mechanism 123, to resume the measurement of the inner diameter of the conduit. Further, in the case where the measurement of the inner diameter of the conduit is to be performed a plurality of times, it is not necessary to discharge the preliminarily introduced fluid each time.

Hereunder, a priming method (hereinafter, simply method where appropriate) for the inner diameter measurement instrument 100 configured as above according to this embodiment, and a method of measuring the inner diameter of the conduit with the inner diameter measurement instrument 100 will be described. This method corresponds to the method of preliminarily filling the inner diameter measurement instrument 100 with the fluid.

To start with, the outline of the method will be described. The method includes introducing the fluid into the balloon element 110 through the first channel member 120 among the first channel member 120 and the second channel member 140 respectively communicating with the balloon element 110, causing the introduced fluid to flow out through the second channel member 140, and closing the second channel member 140 when the balloon element 110, the first channel member 120, and the second channel member 140 are filled with the fluid. The first channel member 120 and the second channel member 140 respectively communicate with the balloon element 110. The respective proximal end portions of the first channel member 120 and the second channel member 140 are connected with a syringe or left open so as to be capable of being closed.

More specifically, the method includes injecting the fluid into the first channel member 120 through the proximal end portion thereof, and causing the fluid to flow out through the proximal end portion of the second channel member, so as to fill the first channel member 120, the balloon element 110, and the second channel member 140 with the fluid. The method further includes closing the proximal end portion of the second channel member 140 filled with the fluid as are the first channel member 120 and the balloon element 110.

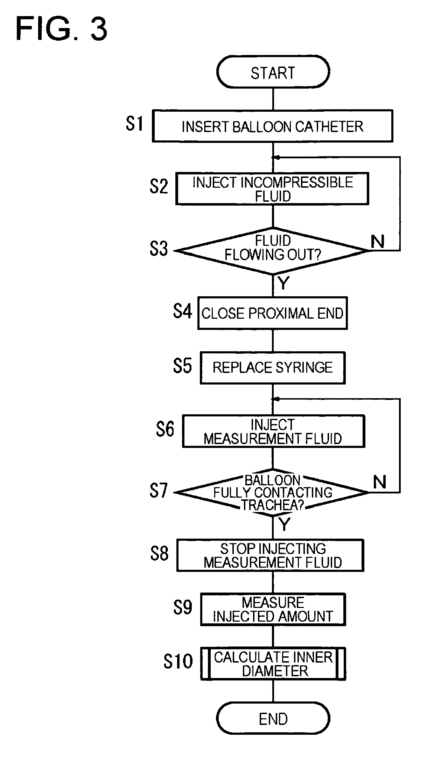

The method will now be described in further details hereunder.
As shown in Fig. 3, the balloon element 110 is inserted in a conduit such as the bronchus of a pneumonectasia patient, to a position where the inner diameter of the conduit is to be measured (step S1). The insertion can be performed by, for example, inserting the portion of the balloon catheter 112 in a forceps channel of a bronchoscope (not shown).

At this stage, the fluid lock mechanism 150 is open. Then the incompressible fluid is injected by the fluid injection mechanism 130 (first syringe 131) through the proximal end portion of the first channel member 120 communicating with the balloon element 110 (step S2).

The fluid thus injected flows into inside the cap 111 and the balloon element 110 through the first channel member 120, and then flows out from the second channel member 140. Accordingly, when the fluid flows out through the proximal end portion of the second channel member 140 (Y at step S3), the proximal end portion of the second channel member 140, now filled with the fluid as are the first channel member 120 and the balloon element 110, is closed by the fluid lock mechanism 150 (step S4). At this stage, the measurement system is preliminarily filled with the fluid under ambient pressure.

The priming process, in which the entirety of the inner diameter measurement instrument 100 is preliminarily filled with the fluid, is thus completed. Since the first syringe 131 has a larger capacity as already stated, the priming process can be quickly completed.

After completion of the priming process, the first syringe 131 is removed from the first channel member 120 and replaced with the second syringe 132 (step S5). Then the measurement fluid is injected by the second syringe 132 which has a smaller capacity, into the first channel member 120 with the fluid lock mechanism 150 set in the closed state (step S6).

During this process, the expanding status of the balloon element 110 in the bronchus can be visually observed through the bronchoscope (not shown), and when the outer circumferential surface of the balloon element 110 makes close contact with the inner circumferential surface of the bronchus (Y at step S7), the injection of the measurement fluid is stopped (step S8). Then the amount of the measurement fluid injected by the second syringe 132 (additionally injected) after the completion of the priming (preliminary filling) by the first syringe 131 is measured with the scale 161 (step S9).

Further, the amount of the measurement fluid is converted into the inner diameter of the conduit which is equal to the outer diameter of the expanded balloon element 110 (step S10). This conversion can be performed, for example, by an inner diameter indicator such as a conversion table (not shown) prepared in advance showing the correlation between the fluid amount and the inner diameter.

With the inner diameter measurement instrument 100 according to this embodiment, the amount of the measurement fluid, injected by the second syringe 132 into the first channel member 120 while the entire measurement system is primed with the fluid, is measured.

Thus, the inner diameter of the conduit can be measured upon injecting the measurement fluid so as to expand the balloon element 110, in the primed state in which the inner diameter measurement instrument 100 is filled with the incompressible fluid.

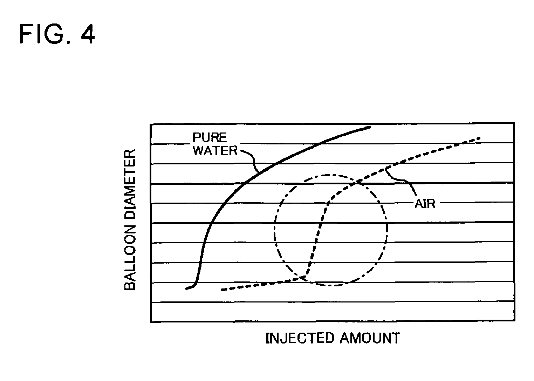

Accordingly, a compressible fluid such as air is restricted from intruding into the measurement system in the stage between the priming process and the inner diameter measurement, and therefore the balloon element 110 can be prevented from abruptly expanding as shown in Fig. 4, which allows the inner diameter of the conduit to be measured safely and accurately.

Further, as shown in Fig. 2, in the inner diameter measurement instrument 100 according to this embodiment the inflow path 121 of the first channel member 120 is smaller in diameter than the outflow path 141 of the second channel member 140. Accordingly, the fluid injection mechanism 132 for injecting the measurement fluid toward the balloon element 110 can be operated with a smaller driving force.

In addition, the outflow path 141 of the second channel member 140 is larger in diameter than the inflow path 121 of the first channel member 120. Such a configuration effectively prevents air bubbles from residing inside the inner diameter measurement instrument 100 in the priming process.

The present invention is in no way limited to this embodiment, but various modifications may be made within the scope of the present invention. For example, the inflow path 121 of the first channel member 120 and the outflow path 141 of the second channel member 140 have different inner diameters in the foregoing embodiment. However, naturally the inner diameters may be the same. In this case, the structure of the inner diameter measurement instrument is simplified (not shown), which results in improved production efficiency.

According to the foregoing embodiment, in the priming process in which the measurement system is preliminarily filled with the fluid, the fluid is injected from the first syringe 131 into the first channel member 120 with the proximal end portion of the second channel member left open. Alternatively, a suction pump (not shown) or the like may be attached to the proximal end portion of the second channel member, so as to positively suck the fluid inside the measurement system with a negative pressure, through the proximal end portion of the second channel member.

According to the foregoing embodiment, the expanding status of the balloon element 110 inside the bronchus is visually observed through a bronchoscope, to check whether the balloon element 110 has been expanded so as to fit the inner diameter of the bronchus.

Alternatively, a pressure gauge (not shown) may be provided in the first channel member 120 or the second channel member 140, so that it can be confirmed whether the balloon element 110 has been expanded so as to fit the inner diameter of the bronchus by measuring the pressure of the fluid.

In addition, though not mandatory, an additional fluid lock mechanism (not shown) may be provided in the first channel member 120, and the additional fluid lock mechanism may be closed when the balloon element 110 is expanded so as to fit the inner diameter of the bronchus by the injection of the measurement fluid from the second syringe 132.

Such an arrangement allows the state in which the bronchus and the balloon element 110 are in close contact with each other to be securely maintained, thereby enabling accurate measurement of the inner diameter of the bronchus which is equal to the outer diameter of the expanded balloon element 110.

Further, according to the foregoing embodiment, the inner diameter indicator such as the conversion table showing the correlation between the amount of the measurement fluid and the outer diameter of the balloon element 110, calibrated in advance, is employed for converting the amount of the fluid into the inner diameter of the conduit.

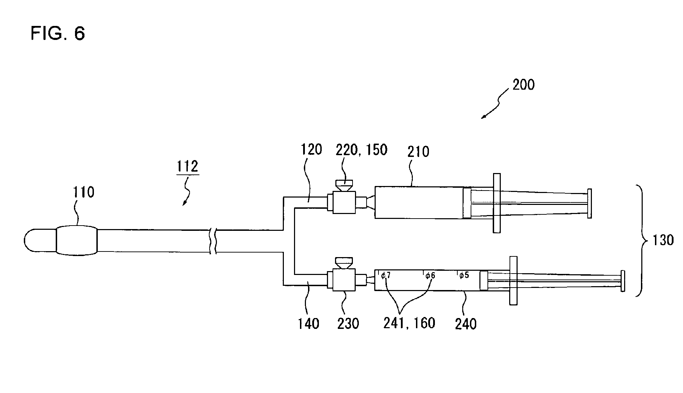

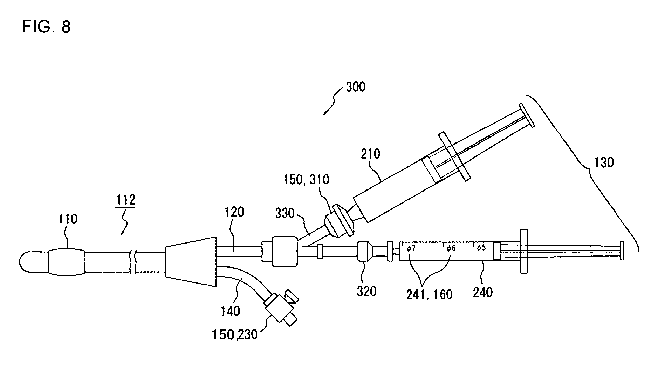

Alternatively, a syringe on which the scale 161 (amount measurement unit 160) directly indicates the inner diameter of the conduit, corresponding to the outer diameter of the expanded balloon element 110, may be employed as the second syringe 132, as in the second or third embodiment to be subsequently described (see conversion scale 241 shown in Figs. 6 and 8).

Further, according to the foregoing embodiment the conduit is exemplified by the bronchus of a human lung. However, the inner diameter measurement instrument 100 according to the first embodiment is broadly applicable to inner diameter measurement of various types of conduit, including those other than human organs.

### Second Embodiment: Second inner diameter measurement instrument

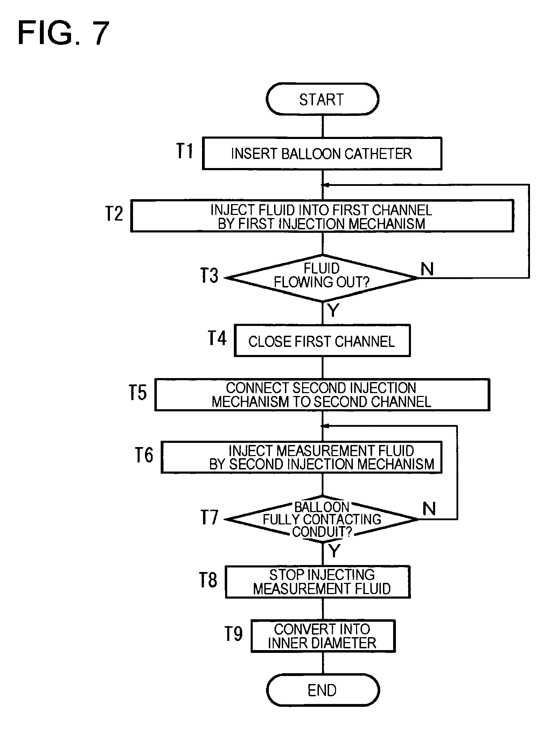

Referring now to Figs. 6 and 7, a second embodiment of the present invention will be described hereunder. Regarding an inner diameter measurement instrument 200 according to this embodiment, the constituents same as those of the inner diameter measurement instrument 100 will be given the same numeral, and the description thereof will not be repeated.

As shown in Fig. 6, in the inner diameter measurement instrument 200 according to this embodiment, the first channel member 120 and the second channel member 140 communicate with the balloon element 110.
The fluid injection mechanism 130 includes a first injection mechanism 210 that injects the fluid into the first channel member 120 through the proximal end portion thereof, and a second injection mechanism 240 removably attached to the proximal end portion of the second channel member 140 so as to inject the measurement fluid.
The fluid lock mechanism 150 includes a first block mechanism 220 that inhibits the reverse flow of the fluid into the first injection mechanism 210, from the first channel member 120 filled with the fluid as are the balloon element 110 and the second channel member 140.
The second injection mechanism 240 serves to inject the measurement fluid into the second channel member 140 through the proximal end portion thereof, with the first block mechanism 220 set to inhibit the reverse flow of the fluid.
The amount measurement unit 160 is used to measure the amount of the measurement fluid injected by the second injection mechanism 240 into the second channel member 140.

As in the first embodiment, the fluid lock mechanism 150 serves to prevent the preliminarily introduced fluid from flowing out from the measurement system because of the additional injection of the measurement fluid. The fluid lock mechanism 150 according to this embodiment includes the first block mechanism 220.

The fluid lock mechanism 150 (first block mechanism 220) according to this embodiment may include a manual valve mechanism for allowing and inhibiting the communication between the first channel member 120 and the first injection mechanism 210, or a one-way valve that allows the fluid to be introduced from the first injection mechanism 210 into the first channel member 120 and inhibits the reverse flow. In this embodiment, the first block mechanism 220 is exemplified by the manual valve mechanism that can be manually operated so as to openably close the first channel member 120.

Though not mandatory, the inner diameter measurement instrument 200 according to this embodiment includes a second block mechanism 230 that openably closes the proximal end portion of the second channel member 140, filled with the fluid as are the first channel member 120 and the balloon element 110. The second injection mechanism 240 containing the measurement fluid is connected to the second block mechanism 230. The second block mechanism 230 is also a manual valve mechanism that can be manually operated so as to openably close the second channel member 140.

The first injection mechanism 210 and the second injection mechanism 240 are each constituted of a syringe for manually injecting the fluid, as the aforementioned fluid injection mechanism 130. However, as in the foregoing embodiment, the first injection mechanism 210 is a syringe of a larger capacity and the second injection mechanism 240 is a syringe of a smaller capacity. The second injection mechanism 240 injects the fluid at smaller injection rate than that of the first injection mechanism 210.

The amount measurement unit 160 according to this embodiment is realized as a conversion scale 241 marked on the outer circumferential surface of the second injection mechanism 240, to serve as the inner diameter indicator showing the inner diameter of the conduit which is equal to the outer diameter of the expanded balloon element 110 obtained on the basis of the measured amount of the measurement fluid.

In the inner diameter measurement instrument 100 according to the first embodiment, the distal end portion of the balloon catheter 112 is closed by the cap 111. However, as shown in Fig. 6, the distal end portion of the balloon catheter 112 of the inner diameter measurement instrument 200 according to this embodiment is closed and formed in a semispherical shape, instead of employing the cap 111.

Hereunder, description will be sequentially given on a priming method (hereinafter, the method) and an inner diameter measurement method to be performed with the inner diameter measurement instrument 200 thus configured according to this embodiment.

The method includes injecting the fluid into the first channel member 120 through the proximal end portion thereof, causing the fluid to flow out through the proximal end portion of the second channel member 140, so as to fill the first channel member 120, the balloon element 110, and the second channel member 140 with the fluid. Then the reverse flow of the fluid through the proximal end portion of the first channel member 120, filled with the fluid as are the balloon element 110 and the second channel member 140, is inhibited.

The method will now be described in further details hereunder. As shown in Fig. 7, first the balloon element 110 is inserted in a conduit to a position where the inner diameter thereof is to be measured, as with the inner diameter measurement instrument 100 (step T1).

At this stage, the second block mechanism 230 is opened and the second injection mechanism 240 is not yet connected. Then the incompressible fluid is injected by the first injection mechanism 210 through the proximal end portion of the first channel member 120 communicating with the balloon element 110 (step T2).

The fluid then flows out through the proximal end portion of the second channel member 140 communicating with the balloon element 110 (Y at step T3). At this stage, the measurement system is preliminarily filled with the fluid under ambient pressure. After confirming such overflow, the first block mechanism 220 is manually operated so as to close the proximal end portion of the first channel member 120. Thus, the reverse flow of the fluid into the first injection mechanism 210 from the first channel member 120, preliminarily filled with the fluid as are the balloon element 110 and the second channel member 140, is inhibited by the first block mechanism 220 (step T4).

Further the second block mechanism 230 for the second channel member 140 is closed, at which point the priming process for the inner diameter measurement instrument 200 is completed. Now, the second injection mechanism 240 is connected to the second block mechanism 230 for the second channel member 140 now closed as above (step T5).

Then the second block mechanism 230 to which the second injection mechanism 240 is now connected is opened, and the measurement fluid is additionally injected at small injection rate by the second injection mechanism 240 connected to the proximal end portion of the second channel member 140, with the first block mechanism 220 set to inhibit the reverse flow of the fluid (step T6).

During this process, the expanding status of the balloon element 110 in the bronchus is visually observed through the bronchoscope (not shown) as in the first embodiment, and when the outer circumferential surface of the balloon element 110 makes close contact with the inner circumferential surface of the bronchus (Y at step T7), the additional injection of the measurement fluid is stopped (step T8). In the case where the close contact between the balloon element 110 and the bronchus has to be maintained, the second block mechanism 230 is closed.

Since the conversion scale 241, showing the outer diameter of the balloon element 110 expanded in proportion to the injected amount of the measurement fluid, is marked on the second injection mechanism 240, the inner diameter of the conduit which is equal to the outer diameter of the expanded balloon element 110 can be measured on the basis of the conversion scale 241 (step T9).

Thus, with the inner diameter measurement instrument 200 according to this embodiment also, the inner diameter of the conduit can be measured upon additionally injecting the measurement fluid so as to expand the balloon element 110, in the primed state in which the inner diameter measurement instrument 200 is preliminarily filled with the incompressible fluid.

Accordingly, a compressible fluid such as air is not involved in the priming process and the inner diameter measurement, and therefore the balloon element 110 can be prevented from abruptly expanding, which allows the inner diameter of the conduit to be measured safely and accurately.

Further, the priming process in which the inner diameter measurement instrument 200 is preliminarily filled with the fluid can be quickly performed by the first injection mechanism 210, and the inner diameter measurement can be precisely performed utilizing the second injection mechanism 240.

In particular, since the additional injection of the measurement fluid by the second injection mechanism 240 is started after completion of the priming process, the amount of the additionally injected measurement fluid uniquely corresponds to the outer diameter of the expanded balloon element 110.

Therefore, the outer diameter of the balloon element 110 expanded in proportion to the amount of the additionally injected measurement fluid can be indicated by the conversion scale 241 marked on the second injection mechanism 240, so that the inner diameter of the conduit, which is equal to the outer diameter of the expanded balloon element 110, can be measured simply by visually checking the conversion scale 241.

The present invention is in no way limited to this embodiment, but various modifications may be made within the scope of the present invention. For example, according to the foregoing embodiment the second block mechanism 230 is employed for openably closing the proximal end portion of the second channel member 140, preliminarily filled with the fluid as are the first channel member 120 and the balloon element 110. However, the second block mechanism 230 may be excluded.

The second block mechanism 230 may be constituted of the reverse flow block mechanism 123 shown in Figs. 5 (a) and 5 (b). More specifically, the second injection mechanism 240 may be connected to the fitting member 129 of the reverse flow block mechanism 123, so as to allow the measurement fluid to be introduced into the second channel member 140 through the one-way valve 124. In this case, the arms 127 are pressed so as to forcibly open up the open end 124a of the one-way valve 124, while preliminarily injecting the fluid. In the process of additionally injecting the measurement fluid from the second injection mechanism 240 connected to the fitting member 129, the initial state of the balloon element 110 can be restored simply by manually opening the one-way valve 124 even though the measurement fluid has been excessively injected, and therefore the measurement can be quickly resumed.

According to the foregoing embodiment, the first block mechanism 220 that inhibits the reverse flow of the fluid into the first injection mechanism 210 from the first channel member 120, preliminarily filled with the fluid as are the balloon element 110 and the second channel member 140, is constituted of the manual valve mechanism that can be manually operated so as to openably close the first channel member 120. Instead, the first block mechanism may be constituted of a one-way valve (not shown) that allows the fluid to be introduced from the first injection mechanism 210 into the first channel member 120 and inhibits the reverse flow.

According to the foregoing embodiment, the distal portion of the balloon catheter 112 is closed in a semispherical shape without employing the cap 111. However, the distal portion of the balloon catheter 112 of the inner diameter measurement instrument 200 may be closed with the cap 111 (not shown).

According to the foregoing embodiment, further, the outer diameter of the balloon element 110 expanded in proportion to the amount of the measurement fluid injected by the second injection mechanism 240 into the measurement system is indicated by the conversion scale 241, so that the inner diameter of the conduit, which is equal to the outer diameter of the expanded balloon element 110, can be measured simply by visually checking the conversion scale 241.

Instead, a syringe only showing a popular scale may be employed as the second injection mechanism 240 (see second syringe 132 shown in Fig. 1), and the inner diameter indicator such as the conversion table showing the correlation between the fluid amount and the inner diameter specified in advance may be looked up so as to convert the amount of the measurement fluid into the inner diameter of the conduit, which is equal to the outer diameter of the expanded balloon element 110, as in the first embodiment.

### Third Embodiment: Third inner diameter measurement instrument

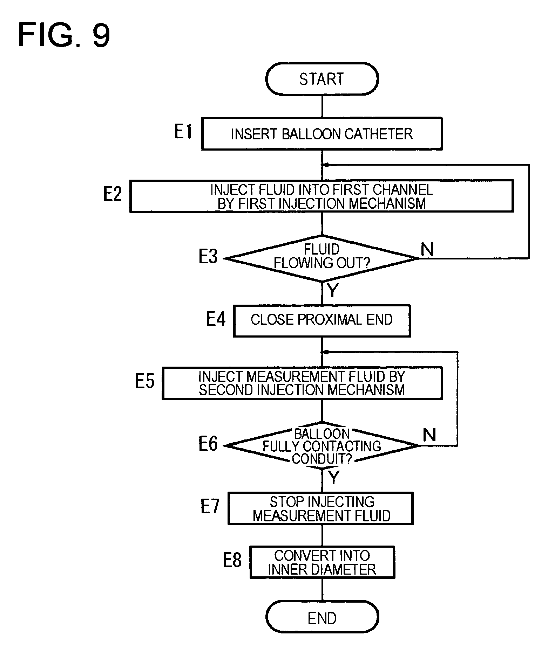

Referring now to Figs. 8 and 9, a third embodiment of the present invention will be described hereunder. Regarding an inner diameter measurement instrument 300 according to this embodiment, the constituents same as those of the inner diameter measurement instruments 100, 200 will be given the same numeral, and the description thereof will not be repeated.

As shown in Fig. 8, in the inner diameter measurement instrument 300 according to this embodiment, the first channel member 120 and the second channel member 140 communicate with the balloon element 110.
The fluid injection mechanism 130 includes the first injection mechanism 210 that injects the fluid into the first channel member 120 through the proximal end portion thereof, and the second injection mechanism 240 that injects the measurement fluid into the first channel member 120 through the proximal end portion thereof.
The fluid lock mechanism 150 includes the second block mechanism 230 openably closes the proximal end portion of the second channel member 140 filled with the fluid as are the balloon element 110 and the first channel member 120. The fluid lock mechanism 150 also includes a first block mechanism 310 that inhibits the reverse flow of the fluid into the first injection mechanism 210 from the first channel member 120 filled with the fluid as are the balloon element 110 and the second channel member 140.
The second injection mechanism 240 serves to inject the measurement fluid into the first channel member 120 through the proximal end portion thereof, with the first block mechanism 310 set to inhibit the reverse flow of the fluid into the first injection mechanism 210.
The amount measurement unit 160 is used to measure the amount of the measurement fluid injected by the second injection mechanism 240 into the first channel member 120.

As in the first and the second embodiment, the fluid lock mechanism 150 serves to prevent the preliminarily introduced fluid from flowing out from the measurement system because of the additional injection of the measurement fluid. The fluid lock mechanism 150 according to this embodiment includes the first block mechanism 310.

The fluid lock mechanism 150 (first block mechanism 310) according to this embodiment is constituted of a one-way valve that allows the fluid to be introduced from the first injection mechanism 210 into the first channel member 120 and inhibits the reverse flow.

The inner diameter measurement instrument 300 according to this embodiment also includes a reverse flow block mechanism 320 that allows the fluid to be introduced from the second injection mechanism 240 into the first channel member 120 and inhibits the reverse flow. The reverse flow block mechanism 320 is constituted of, for example, a duckbill valve that can be manually operated so as to allow the reverse flow.

The first injection mechanism 210 and the second injection mechanism 240 are both connected to the first channel member 120 via a Y-connector 330, an example of two-way connectors. In the inner diameter measurement instrument 300 according to this embodiment also, the amount measurement unit 160 is realized as the conversion scale 241 marked on the outer circumferential surface of the second injection mechanism 240, to serve as the inner diameter indicator showing the inner diameter of the conduit which is equal to the outer diameter of the expanded balloon element 110 obtained on the basis of the measured amount of the measurement fluid.

The inner diameter measurement instrument 300 according to this embodiment includes the second block mechanism 230 that openably closes the proximal end portion of the second channel member 140, filled with the fluid as are the first channel member 120 and the balloon element 110. The second block mechanism 230 is a manual valve mechanism that can be manually operated so as to openably close the second channel member 140.

Hereunder, description will be sequentially given on a priming method (hereinafter, the method) and an inner diameter measurement method to be performed with the inner diameter measurement instrument 300 thus configured according to this embodiment.

The method is applicable to the case where the proximal end portion of the first channel member 120 is branched into a plurality of flow paths, and includes injecting the fluid into the balloon element 110 through one of the flow paths so as to fill the first channel member 120, the balloon element 110, and the second channel member 140 with the fluid. Then the reverse flow of the fluid through the one of the flow paths of the first channel member 120, filled with the fluid as are the balloon element 110 and the second channel member 140, is inhibited, and the proximal end portion of the second channel member 140 filled with the fluid as are the balloon element 110 and the first channel member 120 is closed.

The method will now be described in further details hereunder. As shown in Fig. 9, first the balloon element 110 is inserted in a conduit to a position where the inner diameter thereof is to be measured, as with the inner diameter measurement instruments 100, 200 (step E1).

At this stage, the second block mechanism 230 is opened. Then the incompressible fluid is injected by the first injection mechanism 210 through the proximal end portion of the first channel member 120 communicating with the balloon element 110 (step E2).

The fluid then flows out through the proximal end portion of the second channel member 140 communicating with the balloon element 110 (Y at step E3). At this stage, the measurement system is preliminarily filled with the fluid under ambient pressure. After confirming such overflow, the second block mechanism 230 for the second channel member 140 is closed (step E4), at which point the priming process for the inner diameter measurement instrument 300 is completed.

Now, the measurement fluid is additionally injected in small portions by the second injection mechanism 240 through the other flow path of the branched first channel member 120, with the first block mechanism 310 set to inhibit the reverse flow through the proximal end portion of the one of the flow paths, to which the first injection mechanism 210 is connected (step E5). During this process, the expanding status of the balloon element 110 in the bronchus is visually observed through the bronchoscope (not shown) as in the foregoing embodiments, and when the outer circumferential surface of the balloon element 110 makes close contact with the inner circumferential surface of the bronchus (Y at step E6), the additional injection of the measurement fluid is stopped (step E7).

Since the conversion scale 241, showing the outer diameter of the balloon element 110 expanded in proportion to the injected amount of the measurement fluid, is marked on the second injection mechanism 240, the inner diameter of the conduit which is equal to the outer diameter of the expanded balloon element 110 can be measured on the basis of the conversion scale 241 (step E8).

Thus, with the inner diameter measurement instrument 300 according to this embodiment also, the inner diameter of the conduit can be measured upon additionally injecting the measurement fluid so as to expand the balloon element 110, in the primed state in which the inner diameter measurement instrument 300 is preliminarily filled with the incompressible fluid.

Accordingly, a compressible fluid such as air is not involved in the priming process and the inner diameter measurement, and therefore the balloon element 110 can be prevented from abruptly expanding, which allows the inner diameter of the conduit to be measured safely and accurately.

Further, the priming process in which the inner diameter measurement instrument 200 is preliminarily filled with the fluid can be quickly performed by the first injection mechanism 210, and the inner diameter measurement can be precisely performed utilizing the second injection mechanism 240.

In particular, since the additional injection of the measurement fluid by the second injection mechanism 240 is started after completion of the priming process, the amount of the additionally injected measurement fluid uniquely corresponds to the outer diameter of the expanded balloon element 110.

Therefore, the outer diameter of the balloon element 110 expanded in proportion to the amount of the additionally injected measurement fluid can be indicated by the conversion scale 241 marked on the second injection mechanism 240, so that the inner diameter of the conduit, which is equal to the outer diameter of the expanded balloon element 110, can be measured simply by visually checking the conversion scale 241.

In addition, since there is no need to mount or remove the members during the entire measurement work, the inner diameter measurement can be easily and hygienically performed.

Further, the first block mechanism 310 employed for inhibiting the reverse flow of the fluid from the first channel member 120 into the first injection mechanism 210 is constituted of the one-way valve that allows the fluid to be introduced from the first injection mechanism 210 into the first channel member 120. Such a configuration eliminates the need to manually operate the first block mechanism 310 when the priming process is completed.

The inner diameter measurement instrument 300 according to this embodiment also includes the reverse flow block mechanism 320 that allows the measurement fluid to be introduced from the second injection mechanism 240 into the first channel member 120 and inhibits the reverse flow. Such a configuration prevents the measurement fluid from flowing backward because of the pressure from the expanding balloon element 110, thereby allowing the state in which the bronchus and the balloon element 110 are in close contact with each other to be maintained.

Further, the reverse flow block mechanism 320 is constituted of the duckbill valve that can be manually operated so as to allow the reverse flow. Therefore, the fluid can be allowed to flow backward from the first channel member 120 into the second injection mechanism 240, if need be. Specifically, it is preferable to employ the reverse flow block mechanism 123 shown in Figs. 5(a) and 5(b), as the reverse flow block mechanism 320. In this case, the second injection mechanism 240 is connected to the fitting member 129 of the reverse flow block mechanism 123, so as to allow the measurement fluid to be introduced into the first channel member 120 through the one-way valve 124. In contrast, during the preliminary injection of the fluid by the first injection mechanism 210, the one-way valve 124 is closed. In the case of the additional injection of the measurement fluid, even though an excessive amount of the measurement fluid has been press-injected, the initial state of the balloon element 110 can be restored simply by manually pressing the arms 127 to open the one-way valve 124, and therefore the measurement can be quickly resumed.

The present invention is in no way limited to this embodiment, but various modifications may be made within the scope of the present invention. For example, according to the foregoing embodiment the first block mechanism 310 that inhibits the reverse flow of the fluid into the first injection mechanism 210, filled with the fluid as are the balloon element 110 and the second channel member 140, is constituted of the one-way valve that allows the fluid to be introduced from the first injection mechanism 210 into the first channel member 120 and inhibits the reverse flow. However, the first block mechanism 310 may be constituted of a manual valve mechanism (not shown) that can be manually operated so as to openably close the first channel member 120.

Likewise, according to the foregoing embodiment the reverse flow block mechanism 320 that allows the fluid to be introduced from the second injection mechanism 240 into the first channel member 120 and inhibits the reverse flow is constituted of the duckbill valve which is a one-way valve. However, the reverse flow block mechanism 320 may be constituted of a simple one-way valve or a manual valve mechanism (not shown).

In the case where the first channel member 120, communicating with the Y-connector 330 and the second injection mechanism 240, can be effectively closed simply by pressing the first channel member 120 with the fingers (not shown), the reverse flow block mechanism 320 may be excluded since it is not an essential constituent.

According to the foregoing embodiment also, the distal end portion of the balloon catheter 112 is closed and formed in a semispherical shape, without employing the cap 111. However, the distal end portion of the balloon catheter 112 of the inner diameter measurement instrument 300 configured as above may be closed with the cap 111 (not shown).

According to the foregoing embodiment, both the first injection mechanism 210 and the second injection mechanism 240 are connected to the first channel member 120 via the Y-connector 330. However, various different branched connectors, such as a T-connector (not shown) may be employed for achieving such a connection.

In the inner diameter measurement instruments 100, 200, and 300 respectively described above, the fluid lock mechanism 150 and the second block mechanism 230 that openably close the proximal end portion of the second channel member 140, filled with the fluid as are the first channel member 120 and the balloon element 110, are constituted of the open/close valve that can be operated with the fingers.

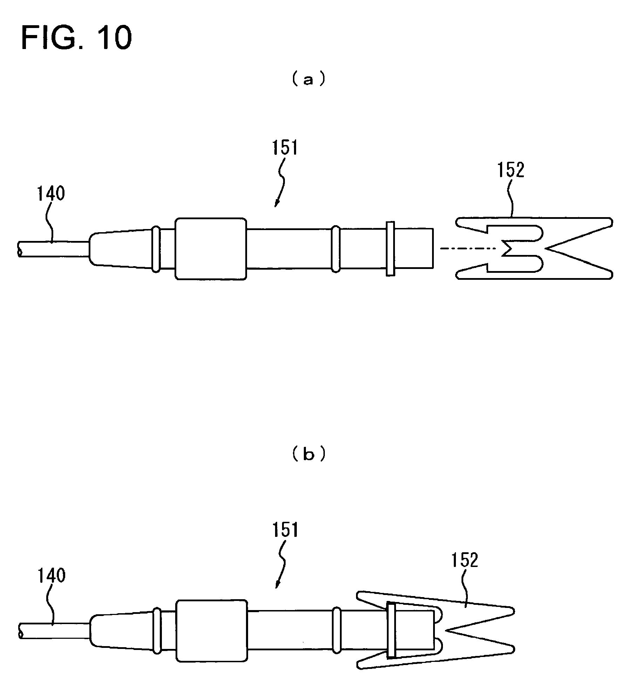

However, the fluid lock mechanism may include, as a first variation shown in Figs. 10(a) and 10(b), a valve mechanism 151 having a valve element (not shown) that closes the proximal end portion of the second channel member 140 and an elastic member (not shown) that biases the valve element to the closed state, and a clamp 152 removably attached to the valve mechanism 151 so as to open the valve element. The valve mechanism 151 is a spring type one-way valve, and when the M-shaped clamp 152 is attached to a proximal end portion of the valve mechanism 151, the elastic member (spring) is compressed so as to maintain the valve element in the open state. When the clamp 152 is not attached to the valve mechanism 151 as shown in Fig. 10(a), the valve element is set to close the flow path by the elastic member located inside the valve mechanism 151. In contrast, when the clamp 152 is attached to the valve mechanism 151 as shown in Fig. 10 (b), the valve element located inside is pressed by the clamp 152 so as to open the flow path.

According to the foregoing embodiment also, the outer diameter of the balloon element 110 expanded in proportion to the amount of the measurement fluid injected by the second injection mechanism 240 is indicated by the conversion scale 241, so that the inner diameter of the conduit, which is equal to the outer diameter of the expanded balloon element 110, can be measured simply by visually checking the conversion scale 241.

Instead, a syringe only showing a popular scale (not shown) may be employed as the second injection mechanism 240, and the inner diameter indicator showing the correlation between the fluid amount and the inner diameter may be looked up so as to convert the amount of the measurement fluid into the inner diameter of the conduit, as in the first embodiment.

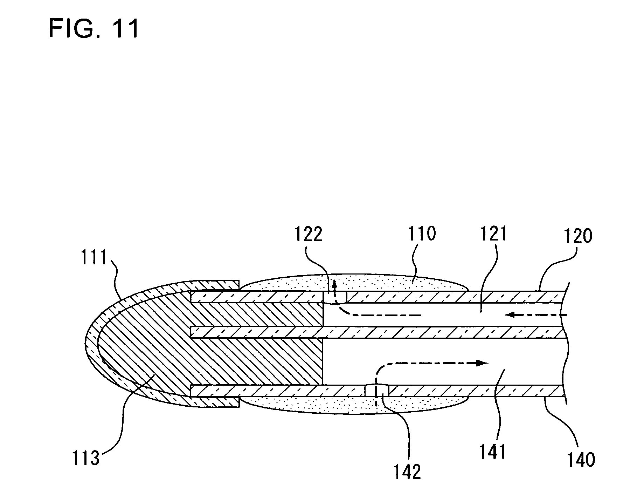

Fig. 11 is a schematic vertical cross-sectional view showing an inner structure of an essential part of an inner diameter measurement instrument according to a second variation. In this variation, the distal end portions of the first channel member 120 and the second channel member 140 are filled with a resin material 113, in a region more distal than one of a first opening and a second opening located at a more distal position than the other (in this variation, orifice 122). The distal portions of the inflow path 121 communicating with the first channel member 120 and the outflow path 141 communicating with the second channel member 140 are both blocked, so that the first channel member 120 and the second channel member 140 can communicate with each other only through the orifices 122, 142 and the balloon element 110. Such a configuration prevents air bubbles from residing inside the balloon element 110 through the preliminary injection (priming) of the fluid through the inflow path 121. In addition, an air vent valve (not shown) may be provided at an appropriate position of the first channel member 120 and the second channel member 140.

This variation is similar to the first embodiment (see Fig. 2) in that the first opening (orifice 122) formed at the distal end portion of the first channel member 120 and the second opening (orifice 142) formed at the distal end portion of the second channel member 140 are located inside the balloon element 110 such that the first opening (orifice 122) and the second opening (orifice 142) communicate with each other through the balloon element 110. On the other hand, this variation is different from the first embodiment in that the first opening (orifice 122), formed in one of the first channel member 120 and the second channel member 140 (in this variation, the first channel member 120) through which the fluid is injected by the fluid injection mechanism 130 into the balloon element 110, is located at a more distal position than the second opening (orifice 142) formed in the other of the first channel member 120 and the second channel member 140(in this variation, the second channel member 140).

Forming thus the orifices 122 and 142 at axially different positions prevents buckling deformation of the distal portion of the inner diameter measurement instrument (measurement system), thereby facilitating the handling of the inner diameter measurement instrument when inserting the same into the conduit. In addition, the orifice 122 is located on the upstream side of the inward flow of the fluid to the balloon element 110 is located at the more distal position than the orifice 142, and the resin material 113 is fluid-tightly loaded on the further distant region adjacent to the orifice 122. Accordingly, air bubbles are suppressed from residing in the vicinity of the orifice 122. On the other hand, in the vicinity of the orifice 142, located on the downstream side of the outward flow of the fluid from the balloon element 110, air bubbles are discharged by a negative pressure generated by the outward flow of the fluid. Therefore, air bubbles are restricted from residing in the space between the orifice 142 and the resin material 113. Consequently, this variation contributes to improving the rigidity of the distal portion of the inner diameter measurement instrument, as well as minimizing residual air bubbles during the preliminary injection of the fluid.

It is a matter of course that the foregoing embodiments and the plurality of variations thereof may be combined unless a conflict of effects is incurred. In addition, although the configurations of the constituents have been specifically described regarding the embodiment and variations, such configurations may be modified in various manners within the scope of the present invention.

The foregoing embodiments encompass the following technical ideas.
(1) An inner diameter measurement instrument that measures an inner diameter of a conduit, including:
   a balloon element inserted to a position where the inner diameter of the conduit is to be measured;
   a first channel member having a distal end portion thereof connected to the balloon element;
   a fluid injection mechanism that injects an incompressible fluid into the first channel member through a proximal end portion thereof;
   a second channel member having a distal end portion thereof connected to the balloon element and configured so as to allow the injected fluid to flow out through a proximal end portion thereof;
   a fluid lock mechanism that openably closes the proximal end portion of the second channel member filled with the fluid as are the first channel member and the balloon element; and
   an amount measurement unit that measures an amount of the fluid injected from the fluid injection mechanism into the first channel member with the fluid lock mechanism set in a closed state.
(2) An inner diameter measurement instrument that measures an inner diameter of a conduit, including:
   a balloon element inserted in the conduit to a position where the inner diameter is to be measured;
   a first channel member having a distal end portion thereof connected to the balloon element;
   a first injection mechanism that injects an incompressible fluid into the first channel member through a proximal end portion thereof;
   a second channel member having a distal end portion thereof connected to the balloon element and configured so as to allow the injected fluid to flow out through a proximal end portion thereof;
   a first block mechanism that inhibits a reverse flow of the fluid into the first injection mechanism from the first channel member filled with the fluid as are the balloon element and the second channel member;
   a second injection mechanism removably connected to a proximal end portion of the second channel member so as to inject the fluid, with the first block mechanism set to inhibit the reverse flow; and
   an amount measurement unit that measures an amount of the fluid injected from the second injection mechanism into the second channel member.
(3) An inner diameter measurement instrument that measures an inner diameter of a conduit, including:
   a balloon element inserted in the conduit to a position where the inner diameter is to be measured;
   a first channel member having a distal end portion thereof connected to the balloon element;
   a first injection mechanism that injects an incompressible fluid into the first channel member through a proximal end portion thereof;
   a second channel member having a distal end portion thereof connected to the balloon element and configured so as to allow the injected fluid to flow out through a proximal end portion thereof;
   a first block mechanism that inhibits a reverse flow of the fluid into the first injection mechanism from the first channel member filled with the fluid as are the balloon element and the second channel member;
   a fluid lock mechanism that openably closes the proximal end portion of the second channel member filled with the fluid as are the balloon element and the first channel member;
   a second injection mechanism that injects the fluid into the first channel member through the proximal end portion thereof, where the reverse flow into the first injection mechanism is inhibited; and
   an amount measurement unit that measures an amount of the fluid injected from the second injection mechanism into the second channel member.
(4) The inner diameter measurement instrument according to (3) above, further including a reverse flow block mechanism that allows the fluid to be introduced from the second injection mechanism into the first channel member, and inhibits the reverse flow.
(5) The inner diameter measurement instrument according to (4) above, in which the reverse flow block mechanism is manually operable so as to allow the reverse flow.
(6) The inner diameter measurement instrument according to any of (2) to (5) above, in which the second injection mechanism injects the fluid at smaller injection rate than that of the first injection mechanism.
(7) The inner diameter measurement instrument according to any of (2) to (6) above, in which the first block mechanism is constituted of a manual valve mechanism manually operable so as to open and close the communication between the first channel member and the first injection mechanism.
(8) The inner diameter measurement instrument according to any of (2) to (6) above, in which the first block mechanism is constituted of a one-way valve that allows the fluid to be introduced from the first injection mechanism into the first channel member and inhibits the reverse flow.
(9) The inner diameter measurement instrument according to any of (1) to (8) above, further including an inner diameter indicator that shows the inner diameter of the conduit corresponding to an outer diameter of the expanded balloon element obtained on the basis of the measured amount of the fluid.
(10) A priming method including filling the inner diameter measurement instrument according to (1) above with the fluid, the method including:
   injecting the incompressible fluid by the fluid injection mechanism into the first channel member having the distal end portion connected to the balloon element through the proximal end portion thereof,
   causing the fluid to flow out through the proximal end portion of the second channel member having the distal end portion connected to the balloon element; and
   setting the fluid lock mechanism so as to close the proximal end portion of the second channel member filled with the fluid as are the first channel member and the balloon element.
(11) A priming method including filling the inner diameter measurement instrument according to (2) above with the fluid, the method including:
   injecting the incompressible fluid by the first injection mechanism into the first channel member having the distal end portion connected to the balloon element through the proximal end portion thereof,
   causing the fluid to flow out through the proximal end portion of the second channel member having the distal end portion connected to the balloon element; and
   setting the first block mechanism so as to inhibit the reverse flow of the fluid into the first injection mechanism from the first channel member filled with the fluid as are the balloon element and the second channel member.
(12) A priming method including filling the inner diameter measurement instrument according to (3) above with the fluid, the method including:
   injecting the incompressible fluid by the first injection mechanism into the first channel member having the distal end portion connected to the balloon element through the proximal end portion thereof,
   causing the fluid to flow out through the proximal end portion of the second channel member having the distal end portion connected to the balloon element;
   setting the first block mechanism so as to inhibit the reverse flow of the fluid into the first injection mechanism from the first channel member filled with the fluid as are the balloon element and the second channel member; and
   setting the fluid lock mechanism so as to close the proximal end portion of the second channel member filled with the fluid as are the balloon element and the first channel member.

This application claims priority based on Japanese Patent Application No.2009-264718 filed on November 20, 2009, the content of which is incorporated hereinto in its entirety.

## Claims

1. An inner diameter measurement instrument to be used for measuring an inner diameter of a conduit, the inner diameter measurement instrument comprising:
a balloon element to be inserted in the conduit to a position where the inner diameter is to be measured;
a first channel member and a second channel member respectively communicating with the balloon element;
a fluid injection mechanism that injects an incompressible fluid into the balloon element through the first channel member or the second channel member;
a fluid lock mechanism that closes one of the first channel member and the second channel member; and
an amount measurement unit that measures an amount of an incompressible measurement fluid additionally injected by the fluid injection mechanism through the other of the first channel member and the second channel member, after the balloon element, the first channel member, and the second channel member are filled with the fluid and the fluid lock mechanism closes the one of the first channel member and the second channel member.

2. The inner diameter measurement instrument according to Claim 1,
wherein the fluid injection mechanism injects the fluid and the measurement fluid into the first channel member through a proximal end portion thereof;
the fluid lock mechanism openably closes a proximal end portion of the second channel member filled with the fluid as are the first channel member and the balloon element; and
the amount measurement unit measures the amount of the measurement fluid injected into the first channel member by the fluid injection mechanism with the fluid lock mechanism set in a closed state.

3. The inner diameter measurement instrument according to Claim 1,
wherein the fluid injection mechanism includes a first injection mechanism that injects the fluid into the first channel member through a proximal end portion thereof, and a second injection mechanism removably connected to a proximal end portion of the second channel member so as to inject the measurement fluid thereinto;
the fluid lock mechanism includes a first block mechanism that inhibits a reverse flow of the fluid into the first injection mechanism from the first channel member filled with the fluid as are the second channel member and the balloon element;
the second injection mechanism injects the measurement fluid through the proximal end portion of the second channel member with the first block mechanism set to inhibit the reverse flow; and
the amount measurement unit measures the amount of the measurement fluid injected by the second injection mechanism into the second channel member.

4. The inner diameter measurement instrument according to Claim 1,
wherein the fluid injection mechanism includes the first injection mechanism that injects the fluid into the first channel member through a proximal end portion thereof and a second injection mechanism that injects the measurement fluid through the proximal end portion of the first channel member;
the fluid lock mechanism openably closes a proximal end portion of the second channel member filled with the fluid as are the balloon element and the first channel member, and inhibits a reverse flow of the fluid into the first injection mechanism from the first channel member filled with the fluid as are the balloon element and the second channel member;
the second injection mechanism injects the measurement fluid through the proximal end portion of the first channel member with a state in which the reverse flow into the first injection mechanism is inhibited; and
the amount measurement unit measures the amount of the measurement fluid injected into the first channel member by the second injection mechanism.

5. The inner diameter measurement instrument according to any of Claims 1 to 4, further comprising a reverse flow block mechanism provided in a flow path of the measurement fluid so as to allow the measurement fluid flowing from the fluid injection mechanism toward the balloon element to be introduced, and so as to inhibit a reverse flow.

6. The inner diameter measurement instrument according to Claim 5,
wherein the reverse flow block mechanism is manually operable so as to allow the reverse flow of the measurement fluid.

7. The inner diameter measurement instrument according to Claim 6,
wherein the reverse flow block mechanism includes a one-way valve to be displaced between an open state that allows the measurement fluid to be introduced and a closed state that inhibits the reverse flow, and a pair of pressers provided on the respective sides of the one-way valve, and the one-way valve is opened so as to allow the reverse flow of the measurement fluid, by pressing the pressers.

8. The inner diameter measurement instrument according to any of Claims 1 to 7,
wherein the fluid injection mechanism injects the measurement fluid at smaller injection rate than that of the fluid.

9. The inner diameter measurement instrument according to Claim 8,
wherein the fluid injection mechanism includes a first syringe that injects the fluid and a second syringe that injects the measurement fluid; and
a scale increment of the second syringe is smaller than a scale increment of the first syringe.

10. The inner diameter measurement instrument according to Claim 2 or 4,
wherein the fluid lock mechanism includes:
a valve mechanism having a valve element that closes the proximal end portion of the second channel member and an elastic member that biases the valve element to the closed state; and
a clamp removably attached to the valve mechanism so as to set the valve element in an open state.

11. The inner diameter measurement instrument according to Claim 3 or 4,
wherein the fluid lock mechanism includes one of a manual valve mechanism that opens and closes communication between the first channel member and the first injection mechanism, and a one-way valve that allows the fluid to be introduced from the first injection mechanism into the first channel member and inhibits the reverse flow.

12. The inner diameter measurement instrument according to any of Claims 1 to 11, further comprising an inner diameter indicator that shows the inner diameter of the conduit corresponding to an outer diameter of the expanded balloon element obtained on the basis of the measured amount of the measurement fluid.

13. The inner diameter measurement instrument according to any of Claims 1 to 12, further comprising a first opening formed at a distal end portion of the first channel member and a second opening formed at a distal end portion of the second channel member,
wherein the first opening and the second opening are located inside the balloon element so as to communicate with each other through the balloon element; and
one of the first opening and the second opening, which is formed in one of the first channel member through which the fluid is injected by the fluid injection mechanism into the balloon element and the second channel member through which the fluid is injected by the fluid injection mechanism into the balloon element, is located at a more distal position than the other formed in the other of the first channel member and the second channel member.

14. The inner diameter measurement instrument according to Claim 13,
wherein a region of the first channel member and the second channel member, on a further distal side of the one of the first opening and the second opening located at the more distal position than the other, is filled with a resin material which is adjacent to the one of the first opening and the second opening.

15. A priming method including introducing an incompressible fluid into a balloon element of an inner diameter measurement instrument, the inner diameter measurement instrument being configured to measures an inner diameter of a conduit by using the balloon element and including a first channel member and a second channel member respectively communicating with the balloon element, the method comprising:
injecting the fluid into the balloon element through the first channel member,
causing the introduced fluid to flow out from the second channel member; and
closing the second channel member when the balloon element, the first channel member, and the second channel member are filled with the fluid.

16. The method according to Claim 15, further comprising:
injecting the fluid into the first channel member through a proximal end portion thereof until the fluid flows out through a proximal end portion of the second channel member, so as to fill the first channel member, the balloon element, and the second channel member with the fluid; and
closing the proximal end portion of the second channel member filled with the fluid as are the first channel member and the balloon element.

17. The method according to Claim 15, further comprising:
injecting the fluid into the first channel member through a proximal end portion thereof until the fluid flows out through a proximal end portion of the second channel member, so as to fill the first channel member, the balloon element, and the second channel member with the fluid; and
inhibiting a reverse flow of the fluid through the proximal end portion of the first channel member filled with the fluid as are the balloon element and the second channel member.

18. The method according to Claim 16, to be performed in the case where the proximal end portion of the first channel member is branched into a plurality of flow paths, the method comprising:
injecting the fluid into the balloon element through one of the flow paths so as to fill the first channel member, the balloon element, and the second channel member with the fluid;
inhibiting a reverse flow of the fluid through the one of the flow paths of the first channel member filled with the fluid as are the balloon element and the second channel member, and closing the proximal end portion of the second channel member filled with the fluid as are the balloon element and the first channel member.
